(19) Europäisches Patentamt
European Patent Office
Office européen des brevets

(11) **EP 1 070 035 B1**

## (12) EUROPÄISCHE PATENTSCHRIFT

(45) Veröffentlichungstag und Bekanntmachung des Hinweises auf die Patenterteilung:
**26.03.2003 Patentblatt 2003/13**

(51) Int Cl.$^7$: **C07C 17/25**, C07C 17/38, C07C 17/383, C07C 21/06

(21) Anmeldenummer: **99908795.0**

(22) Anmeldetag: **08.01.1999**

(86) Internationale Anmeldenummer:
**PCT/EP99/00061**

(87) Internationale Veröffentlichungsnummer:
**WO 99/051554 (14.10.1999 Gazette 1999/41)**

(54) **VERFAHREN ZUR AUFARBEITUNG EINES SPALTGASES AUS DER SPALTUNG VON 1,2-DICHLORETHAN**

METHOD FOR PROCESSING A PRODUCT GAS ARISING FROM THE BREAKDOWN OF 1,2-DICHLOROETHANE

PROCEDE DE RETRAITEMENT D'UN GAZ DE CRAQUAGE ISSU DU CRAQUAGE DE 1,2-DICHLORETHANE

(84) Benannte Vertragsstaaten:
**BE DE FR GB IT NL SE**

(30) Priorität: **07.04.1998 DE 19815446**

(43) Veröffentlichungstag der Anmeldung:
**24.01.2001 Patentblatt 2001/04**

(73) Patentinhaber: **Uhde GmbH**
**44141 Dortmund (DE)**

(72) Erfinder: **SEIDELBACH, Friedrich**
**D-65207 Wiesbaden (DE)**

(74) Vertreter: **Dabringhaus, Walter, Dipl.-Ing. Patentanwälte**
**Meinke, Dabringhaus und Partner,**
**Rosa-Luxemburg-Strasse 18**
**44141 Dortmund (DE)**

(56) Entgegenhaltungen:
DE-A- 4 132 761      US-A- 3 468 967
US-A- 3 655 787      US-A- 4 822 932
US-A- 5 507 921

**Beschreibung**

Die Erfindung betrifft ein Verfahren zur Aufarbeitung eines Spaltgases, welches bei der Spaltung von 1,2-Dichlorethan zu Vinylchlorid anfällt.

In Verfahren zur Herstellung von Vinylchlorid durch unvollständige Spaltung von 1,2-Dichlorethan (EDC) wird üblicherweise das eingesetzte EDC im ersten Schritt verdampft, dann in einem zweiten Schritt der gebildete Dampf bei relativ hoher Temperatur pyrolytisch gespalten, weiter werden in einem dritten Schritt aus dem im zweiten Schritt erzeugten heißen Spaltgas die mitgeführten Feststoffe abgetrennt und nachfolgend das gereinigte Spaltgas einer destillativen Aufarbeitung zugeführt.

Als Hauptprodukte der im zweiten Verfahrensschritt vorgenommenen Spaltung des EDC entstehen Chlorwasserstoff (HCL) und Vinylchlorid (VCM):

$$C_2H_4CL_2 + \text{Wärme} \rightarrow HCL + C_2H_3CL$$

An Nebenprodukten fallen in Spuren Ruß, chlorierte und ungesättigte Kohlenwasserstoffe sowie Benzol an. Um die Bildung dieser unerwünschten Nebenprodukte einzuschränken, wird die Temperatur der Spaltung auf einem Niveau gehalten, die zu einer unvollständigen Umsetzung des EDC führt. Daher enthält das im zweiten Verfahrensschritt durch Spaltung erzeugte heiße Spaltgas zusätzlich zu den Hauptprodukten Chlorwasserstoff (HCL) und Vinylchlorid (VCM) sowie den genannten Nebenprodukten auch noch das nicht umgesetzte 1,2-Dichlorethan (EDC).

Die Spaltung des EDC zu VCM ist ein endotherm ablaufender Vorgang. Sie erfolgt in der Gasphase in Form der Pyrolyse. Technisch geschieht die Pyrolyse katalysatorfrei unter hohem Druck von 1 bis 3 MPa und bei einer Temperatur von 450 bis 600 °C. Es wird aber auch an katalytischen Verfahren gearbeitet, die es erlauben, die Pyrolyse bei niedrigerem Druck und niedrigerer Temperatur durchzuführen. Das mittels Pyrolyse erzeugte heiße Spaltgas fällt bei Pyrolysetemperatur an. Es ist so zu konditionieren, daß es eine für die eigentliche Stofftrennung geeignete Form annimmt.

Vor der eigentlichen Stofftrennung des Spaltgases wird daher die Spaltgas-Wärme in einem oder mehreren Wärmetauschern wirtschaftlich genutzt. Dabei sinkt im Fall der katalysatorfreien Pyrolyse die Temperatur des Spaltgases von 480 bis 540 °C auf etwa 180 bis 280 °C ab.

In einer von der Uhde GmbH im Juni 1995 herausgegebenen Firmendruckschrift mit dem Titel "Vinyl chloride plants / Hoechst process" wird das bisher übliche Verfahren zur Aufarbeitung des Spaltgases dargestellt.

In dem auf Seite 11 dieser Firmenschrift wiedergegebenen Verfahrensfließbild ist gezeigt, wie das vorgekühlte Spaltgas dann in einer Quenche weiter gekühlt und teilkondensiert wird. Dazu wird ein gekühltes Spaltgaskondensat oben in der Quenchzone aufgegeben. In der Quenchzone entstehen aus dem Spaltgas und dem oben aufgegebenen Spaltgaskondensat zwei Produkt (VCM) enthaltende Abzüge:

- aus dem am Boden der Quenchzone abfließenden Ablauf wird der Sumpfablauf gebildet;
- die am Kopf der Quenchzone abströmenden Brüden bilden das Quenchgas.

In der Quenchzone geschieht lediglich eine Teilkondensation des Spaltgases. Daher enthält die aus der Quenchzone unten ablaufende Flüssigkeit, der sogenannte Sumpfablauf, als Hauptbestandteile 1,2-Dichlorethan, Vinylchlorid und Hochsieder sowie die Feststoffe, also Ruß und/oder Koks.

Das am Kopf der Quenchzone als Brüden abströmende Quenchgas enthält als Hauptbestandteile Chlorwasserstoff, Vinylchlorid und 1,2-Dichlorethan. Es ist im großen und ganzen frei von den Feststoffen, also frei von Ruß und/oder Koks.

Das als Quenchflüssigkeit eingesetzte Spaltgaskondensat wird üblicherweise als Teilstrom von dem kondensierten Quenchgas abgezweigt, wodurch ein Kreislauf der Quenchflüssigkeit eintritt.

Der Sumpfablauf und das teilweise kondensierte Quenchgas werden anschließend weiter aufgearbeitet.

In EP-0 276 775-B1 werden für die Temperatur der katalysatorfreien Pyrolyse 450 bis 550 °C und Pyrolysedrücke von 0,5 bis 3 MPa, bevorzugt aber 1,6 bis 2,6 MPa sowie schonende Maßnahmen der Stoffstromführung genannt.

In DE-23 13 037-C3 wird eine schonende Anordnung der Verdampfung beschrieben.

Weil es sich als vorteilhaft erwiesen hat, die vom Spaltgas mitgeführten Feststoffpartikel vor der destillativen Aufarbeitung des Spaltgases von ihm abzutrennen, geschieht dies üblicherweise zusammen mit seiner Kühlung und Teilkondensation in der Quenchzone. Im allgemeinen bewirkt eine einfach gestaltete Quenchvorrichtung, die im wesentlichen aus einem vertikalen Behälter und einer Vorrichtung zum Verdüsen von Quenchflüssigkeit im Inneren des Behälters besteht, eine fast vollständige Reinigung des Spaltgases. Die abgereinigten Feststoffe fallen im Sumpf der Quenchzone an.

In der US-5,558,746 wird eine aufwendiger gestaltete Quenchkolonne mit Böden beschrieben, bei der die Feststoffe ebenfalls zusammen mit dem Sumpfablauf ausgeschleust und anschließend abgetrennt werden. Gleichzeitig ist die üblicherweise in einer externen Vorrichtung durchgeführte Kondensation des Spaltgases in die Quenchkolonne integriert.

Die Pyrolyse von 1,2-Dichlorethan ist bekanntermaßen stark endotherm und durch den Verbrauch hoher Wärme-Energiemengen gekennzeichnet.

Es hat deshalb nicht an Vorschlägen für eine möglichst weitgehende Rückgewinnung der in dem heißen Spaltgas aus der Pyrolyse enthaltenen Wärme mittels Wärmeübertragung auf andere Medien gefehlt:

In EP-0 276 775-B1 werden vier Varianten für die Nutzung des Spaltgas-Wärmeinhaltes für Kombinationen von Vorwärmen, Verdampfen und Überhitzen des EDC-Einsatzes in die Pyrolyse sowie für das Erzeugen von Wasserdampf angegeben.

In DE-31 47 310-C2 wird die Nutzung des Wärmeinhaltes des Spaltgases für die Wasserdampferzeugung oder für die Beheizung von Sumpfumlaufverdampfern in der destillativen Aufarbeitung der VCM-Herstellung angegeben.

In EP-0 180 995-B2 wird der in einer Quenchkolonne von Feststoffen befreite Produktstrom zur Dampferzeugung und Anwärmung von EDC-Einsatz zur Pyrolyse verwendet.

Den vorstehend genannten Verfahrens-Varianten ist gemeinsam, daß das pyrolytisch erzeugte heiße Spaltgas entweder weitgehend bis in die Nähe des Taupunktes abgekühlt oder gar am Taupunkt kondensiert wird und deshalb in der nachfolgenden destillativen Aufarbeitung wieder aufgeheizt werden muß. Auch bei umfassender Anwendung von vorstehend beschriebenen Wärmerückgewinnungs-Maßnahmen verbleibt einerseits ein beachtlicher Rest an nicht ausgenutzter, durch Kühlung an die Umgebung verlorener Wärme und andererseits ein Defizit an erforderlichenfalls wieder aufzubringender Wärme.

In der oben bereits genannten, von der Uhde GmbH im Juni 1995 herausgegebenen Firmendruckschrift mit dem Titel "Vinyl chloride plants / Hoechst process" wird das bisher übliche Verfahren zur destillativen Aufarbeitung des Spaltgases dargestellt. In dem auf Seite 13 dieser Firmenschrift unter der Überschrift "VCM distillation" wiedergegebenen Verfahrensfließbild ist gezeigt, wie das aus der Quenchzone abgezogene, von Feststoffen befreite Spaltgas dann zu VCM aufgearbeitet wird, wobei die destillative Aufarbeitung des pyrolytisch erzeugten Spaltgases in der Hauptsache auf die eigentliche stoffliche Trennung eines Dreistoffsystemes mit den drei Hauptkomponenten HCL, VCM und nicht umgesetztes EDC hin ausgerichtet ist.

Dreistoffsysteme benötigen zu ihrer Trennung eine vergleichsweise aufwendige Trennapparatur. Das vorliegende Dreistoffsystem besitzt außerdem noch einen sehr weiten Siedebereich, wodurch die Trennaufgabe energetisch aufwendigere Lösungen erfordert als ein Gemisch mit engerem Siedebereich.

Zwischen den Siedetemperaturen von HCL (minus 85° C) und EDC (plus 83,5° C) besteht ein Temperaturunterschied von 168,5° C. Wegen der sehr tiefen Siedetemperatur des Chlorwasserstoffes (HCL) muß zur Abtrennung von HCL bevorzugt mit kälteliefernden Kühlmedien gekühlt werden. Technisch wird die Abtrennung von HCL beispielsweise bei 1,3 MPa absolut und -24° C durchgeführt. Die technische Erzeugung von Kälte abgebenden Kühlmedien ist wesentlich aufwendiger und auch kostspieliger als die Bereitstellung eines Kühlmediums, das die Wärmeabfuhr auf dem Temperaturniveau der Umgebung zu bewerkstelligen hat.

Bei den bisherigen Verfahren der destillativen Aufarbeitung, wie sie in den deutschen Patentschriften DE-12 50 426, DE-19 10 854-C3 und DE-43 42 042-A1 beschrieben sind, wird in der ersten Destillationskolonne der Chlorwasserstoff (HCL) über Kopf abgetrennt. Um den Bedarf an Kälte auf ein wirtschaftlich sinnvolles Maß zu begrenzen, ist es unumgänglich, die pyrolytisch erzeugten Spaltgase weitgehend kondensiert in die erste Destillationskolonne einzuspeisen.

Weiterhin enthält beim bisherigen in der oben genannten Firmendruckschrift der Uhde GmbH gezeigten Verfahren der aus der ersten Destillationskolonne abgezogene Sumpfablauf im wesentlichen das als Produkt bewertete VCM sowie das nicht umgesetzte EDC. Dieser Sumpfablauf wird auf die zweite Kolonne aufgegeben. In der zweiten Kolonne wird er in nicht umgesetztes EDC und VCM aufgetrennt. Das über Kopf der zweiten Kolonne abdestillierte VCM enthält jedoch nicht vermeidbare Anteile an HCL, die in einer dritten Kolonne unter Einsatz zusätzlicher Energiezufuhr abgetrennt werden müssen.

Die Ursache für das Auftreten von HCL in den am Kopf der zweiten Destillationskolonne abgezogenen Brüden ist bei dem bisherigen Verfahren vor allem anderen in der thermischen Instabilität des EDC begründet. Durch geeignete, hinreichend empfindliche Analyse-Verfahren lassen sich schon bei Temperaturen ab 100 °C die Zersetzungsprodukte HCL und VCM in reinem EDC nachweisen. Mit steigender Temperatur und zunehmender Verweilzeit nimmt außerdem noch die Menge an gebildetem HCL und VCM zu.

Daher besitzt das bisherige Verfahren noch ein erhebliches Potential für weitere Verbesserungen. Zum einen erlaubt die bisherige Destillationsreihenfolge mit Abtrennung des HCL in der ersten Stufe keine weitergehende Ausnutzung der im pyroltisch erzeugten Spaltgas enthaltenen Wärme, da zur Minimierung des Bedarfes an Kälteenergie das Spaltgas weitgehend kondensiert werden muß.

Zum anderen erfordert die Abtrennung des VCM von allen höhersiedenden Komponenten in der zweiten Stufe einen erheblichen Aufwand an Energie, verbunden mit der Notwendigkeit, aufgrund der thermischen Instabilität des EDC neugebildetes HCL in einer zusätzlichen Reinigungsstufe abzutrennen.

Hier setzt nun das erfindungsgemäße Verfahren an und stellt sich die Aufgabe, das bestehende Verfahren dadurch zu verbessern, daß bereits in der ersten Stufe der Destillationsreihenfolge HCL und VCM von allen höhersiedenden Komponenten abgetrennt werden und der unvermeidbare Einsatz an Kältemittel erst in der nachfolgenden Stufe zur Abtrennung des HCL erfolgt.

Dadurch wird es erfindungsgemäß möglich, insbesondere die Energieverluste, die üblicherweise durch weitgehende Kondensation des Quenchgases und sein Wiederaufheizen zwecks destillativer Trennung entstehen, zu vermeiden sowie durch Kreislaufführung des nicht umgesetzten EDC auf höherem Temperaturniveau den ursprünglichen Umsatz der pyrolytischen Spaltung von EDC nachträglich zu erhöhen.

Hierzu sieht die Erfindung ein Verfahren zur Aufarbeitung des Spaltgases vor, bei dem das eingesetzte Spaltgas aus der Pyrolyse von 1,2-Dichlorethan (EDC) entstanden ist, das Spaltgas in seine Hauptkomponenten Chlorwasserstoff (HCL), Vinylchlorid (VCM) und nicht umgesetztes 1,2-Dichlorethan (EDC) zerlegt wird und diese Hauptkomponenten in weitgehend reiner Form anfallen, wobei im ersten Schritt der Aufarbeitung des Spaltgases von diesem die Feststoffe in einer Quenchzone abgetrennt werden und das Verfahren dadurch gekennzeichnet ist, daß:

- in einer der Quenchzone (1) folgenden Verstärkungszone (2) das gesamte HCl und VCM als Kopfprodukt abgezogen und einer Destillationszone (3) zur Trennung zugeführt wird;

- ein Teil des Sumpfproduktes der Verstärkungszone (2) einer Abtriebszone (4) zugeführt (36) wird, der andere Teil des Sumpfproduktes der Verstärkungszone (2) als ein mit EDC angereichertes, von Feststoffen freies Spaltgaskondensat als Quenchflüssigkeit (37) in der Quenchzone (1) durch Rückführung eingesetzt wird, und daß

- der Kopfprodukt der Abtriebszone (4) rollständig in die Verstärkungszone (2) zurückgeführt wird.

Ausgestaltungen der Erfindung ergeben sich aus den weiteren Ansprüchen.

In der bevorzugten Ausgestaltung betrifft die Erfindung ein Verfahren zur Aufarbeitung des Spaltgases, welches bei der nicht katalytischen thermischen Spaltung von 1,2-Dichlorethan zu Vinylchlorid mit einer Temperatur von 480 bis 540 °C und einem Druck von 0,5 MPa bis 3 MPa anfällt und ggf. in einer Wärmerückgewinnung auf eine Temperatur von 180 bis 280 °C gekühlt und mit dieser Temperatur in eine Quenchzone geleitet wird, in der es mit EDC angereichertem Spaltgaskondensat gekühlt und gewaschen wird, wobei das Verfahren dadurch gekennzeichnet ist, daß man:

- 1 bis 20 Gew.-% bezogen auf die Menge an eingesetztem Spaltgas im Sumpf der Quenchzone flüssig als feststoffbeladener Sumpfablauf entnimmt,
- die verbleibende Menge (eingesetztes Spaltgas plus Quenchflüssigkeit abzüglich Sumpfablauf) am Kopf der Quenchzone gasförmig als gereinigtes Quenchgas entnimmt,
- das hierbei erhaltene Quenchgas unmittelbar unten in eine Verstärkungszone einleitet und das Quenchgas darin in ein Destillat, welches frei von höher als VCM siedenden Komponenten ist, und einen mit EDC angereicherten Sumpf auftrennt,

- den in der Verstärkungszone anfallenden Sumpf ableitet und so das besagte, mit 1,2-Dichlorethan (EDC) angereicherte, von Feststoffen freie Spaltgaskondensat gewinnt und es in zwei Teilströme auftrennt und als den einen Teilstrom den Vorlauf für die Abtriebszone erhält und als den anderen Teilstrom jenes EDC-angereicherte, feststofffreie Spaltgaskondensat erhält, welches in die Quenchzone zurückgeleitet wird, um darin mit ihm als Kühl- und Waschmittel das aufzuarbeitende Spaltgas zu behandeln;

- und daß man das Destillat und den Vorlauf getrennt den weiteren destillativen Aufarbeitungen zuführt

- und den aus der Quenchzone kommenden, mit dem auszuschleusenden Feststoff beladenen Sumpfablauf in eine andere Aufarbeitung einleitet.

Beim erfindungsgemäßen Verfahren werden das für die eine weitere destillative Aufarbeitung vorgesehene Destillat und der für die andere weitere destillative Aufarbeitung vorgesehene Vorlauf frei von irgendeiner Feststoffbeladung gewonnen. Damit stellt sich auch beim erfindungsgemäßen Verfahren der dem bisherigen Verfahren eigene beachtliche Vorteil ein. Es ist nämlich von Vorteil, wenn es vermieden werden kann, daß die aus dem Spaltgas herrührenden Feststoffe, wie Ruß und/oder Koks, in die Apparaturen der weiteren destillativen Aufarbeitungen gelangen und sich dort ablagern können. Auf derart einfache Weise wird dem Auftreten von Verstopfungen im praktischen Betrieb der Apparaturen entgegengewirkt.

Beim bisherigen Verfahren fallen nämlich, wie vorstehend bereits beschrieben, die abgereinigten Feststoffe ausschließlich im Sumpf der Quenchzone an, wodurch eine einfache Aufkonzentrierung und Entsorgung der Feststoffsuspension geschehen kann.

Besonders überraschend hat sich nun beim erfindungsgemäßen Verfahren herausgestellt, daß das in die Verstärkungszone eintretende Quenchgas bei einer EDC-Spaltrate von mindestens 50 % in der vorgeschalteten Pyrolyse so zusammengesetzt ist, daß es bei einem absoluten Druck von mindestens 1,9 MPa über dem obersten Boden der Verstärkungszone genügt, den Rückflußkühler der Verstärkungszone lediglich mit einem bei Umgebungstemperatur vorlaufenden Kühlwasser zu beaufschlagen, um einen kräftigen Rückfluß in die Verstärkungszone zu gewinnen. Weiterhin hat sich bei dem erfindungsgemäßen Verfahren überraschend herausgestellt, daß mit mindestens 25 theoretischen Trennstufen für die Verstärkungszone und einem Rückflußverhältnis von 1,5 bis 2,5 erstens Kopfdampf-, Rückfluß- und Destillat-Ströme erhalten werden, die frei von allen höher als VCM siedenden Komponenten, wie z.B. Chloropren oder EDC sind, und daß zweitens der Ablauf vom untersten Boden der Verstärkungszone auf über 80 Gew.-% mit EDC angereichert ist, weil das EDC diejenige der Hauptkomponenten des Dreistoffgemisches ist, die den höchsten Siedepunkt aufweist.

Erfindungsgemäß ergibt sich, daß die thermische Instabilität des EDC bei dem einen Teilstrom, der den Vorlauf der Abtriebszone bildet, unschädlich in bezug auf die Reinheitsanforderungen des Produktes VCM sind. Sofern sich infolge der thermischen Instabilität in der Abtriebszone HCL und VCM als Zersetzungsprodukte bilden, werden sie am Kopf der Abtriebszone anfallen und erfindungsgemäß von dort in die Verstärkungszone rückgeleitet. Eine schonende Ausführung der Destillation, wie sie im bisherigen Verfahren in der zweiten Stufe der Destillation angebracht war, ist erfindungsgemäß nicht mehr erforderlich. Insbesondere kann im Sumpf der Abtriebszone unbeschadet Druck und Temperatur erhöht werden. In der Verstärkungszone werden die Zersetzungsprodukte wiederum am Kopf ausgeschleust und gemeinsam in die Destillationszone übergeleitet. In der Destillationszone kann jedoch keine thermische Instabilität zur Wirkung kommen, weil die Destillationszone weit unterhalb von 100° C in allen ihren Trennstufen betrieben wird.

Deshalb sieht eine Ausgestaltung des erfindungsgemäßen Verfahrens vor, daß die destillative Aufarbeitung des Vorlaufes in der Abtriebszone geschieht, mit dem darin gebildeten Sumpfprodukt das angereichert rückgewonnene 1,2-Dichlorethan (EDC) abgezogen wird und das darin gebildete Kopfprodukt in die Verstärkungszone zurückgeleitet wird.

Deshalb sieht eine andere Ausgestaltung des erfindungsgemäßen Verfahrens vor, daß die destillative Aufarbeitung des Destillates in der Destillationszone geschieht, mit dem darin gebildeten Sumpfprodukt das abgetrennte Vinylchlorid (VCM) hoch angereichert abgezogen wird und mit dem darin gebildeten Kopfprodukt der Chlorwasserstoff (HCL) angereichert rückgewonnen wird. Aus Gründen eines sicheren Betriebes, insbesondere zum Abfangen eines HCL-Durchbruches im VCM-Produkt bei nicht optimalem Arbeiten der Kolonne, ist eine gesonderte Feinreinigungszone wie beim bisherigen Verfahren sinnvoll.

Das erfindungsgemäße Verfahren nutzt den Wärmeinhalt des pyrolytisch erzeugten Spaltgases nun besonders vorteilhaft fast vollständig aus, indem das Quenchgas direkt unten in die Verstärkungskolonne als destillativ zu behandelnder Dampf eingespeist wird. Weiterhin hat die erfindungsgemäße Zusammenschaltung der Quenchzone mit der neuartig gestalteten Destillationssequenz der Aufarbeitung die verweilzeiterhöhte Kreislaufführung des nicht umgesetzten EDC bei höheren Temperaturen zur Folge, weshalb in Verbindung mit der thermischen Instabilität des EDC es zu einer unerwarteten Ausbeutesteigerung ohne Erfordernis einer zusätzlichen Reinigungsstufe kommt.

Eine zweckmäßige Ausgestaltung des Verfahrens kann aus folgenden Schritten bestehen, daß:

- erstens das zu trennende Gasgemisch mit Temperaturen von 85 bis 540 °C und mit einem Druck von 0,1 MPa bis 3,0 MPa vorliegen kann,

- zweitens das zu trennende Gasgemisch vor Eintritt in die erste Destillationskolonne gasförmig unter die Einbauten einer Verstärkungszone geleitet wird,

- drittens am Kopf der Verstärkungszone ein Gemisch aus HCL,VCM und leichter als VCM siedenden Komponenten abgezogen und als Zulauf zu einer Destillationszone geführt wird,

- viertens als Sumpfprodukt der Verstärkungszone ein Gemisch anfällt, welches aus EDC als Hauptkomponente und aus HCL,VCM und höher als VCM siedenden Komponenten als Nebenkomponenten besteht und welches als Teilstrom, falls Quenchflüssigkeit für eine zuvor zu erfolgende Abtrennung von Feststoffen aus dem eingesetzten Gasgemisch benötigt wird, oder als Gesamtstrom zu einer Abtriebszone geleitet wird,

- fünftens das Kopfprodukt der Verstärkungszone in der Destillationszone in hochreines VCM, welches als Sumpfprodukt der Destillationszone anfällt, und in weitgehend reines HCL, welches aus HCL und leichter als VCM siedenden Komponenten besteht und am Kopf der Destillationszone anfällt,aufgetrennt wird und

- sechstens in der Abtriebszone das aus dem Sumpf der Verstärkungszone zulaufende Gemisch aufgetrennt wird, wobei im Sumpf der Abtriebszone weitgehend reines EDC mit allen höher als VCM siedenden Komponenten und nur sehr geringem Gehalt an HCL und VCM abgezogen wird, während am Kopf der Abtriebszone ein Gemisch gewonnen wird, welches aus EDC und im Zulauf eingetragenem HCL und VCM sowie durch die thermische Instabilität von EDC neu gebildetem HCL und VCM besteht, und zwecks weiterer Aufarbeitung in die Verstärkungszone zurückgeführt wird.

Das Verfahren kann sich auch dadurch auszeichnen, daß ein in die Verstärkungszone eingesetztes, von Feststoffen befreites, aber mit relativ niedrigem Druck ausgestattetes Gasgemisch mit einer Kompressionseinheit auf einen Druck gebracht wird, der ein wirtschaftliches Arbeiten der Verstärkungszone sichert.

Vorteilhaft kann es sein, wenn die Abtriebszone sowohl bei niedrigerem als auch bei höherem Druck als dem in der Verstärkungszone verwendeten Druck betrieben wird. Im Fall eines niedrigeren Druckes in der Abtriebszone kann die Druckerhöhung des zur Verstärkungszone rückgeführten Kopfproduktes der Abtriebszone entweder durch Totalkondensation dieses Kopfproduktes mit anschließender Druckerhöhung über eine Pumpe oder im gasförmigen Zustand mit einer Kompressionseinheit erfolgen. Im Fall eines höheren Druckes in der Abtriebszone erfolgt die Druckanpassung durch eine Pumpe in dem aus dem Sumpf der Verstärkungszone stammenden Zulauf zur Abtriebszone.

Das Verfahren kann sich auch dadurch auszeichnen, daß einer oder mehrere Sumpfumlaufverdampfer der Destillationszone mit dem Sumpfprodukt der Abtriebszone beheizt werden, wobei ebenfalls vorgesehen sein kann, daß das Sumpfprodukt der Destillationszone über eine wahlweise zuschaltbare Feinreinigungszone geleitet wird, wobei ein hochreiner Produktstrom im Sumpf der Feinreinigungszone abgezogen wird und das Kopfprodukt der Feinreinigungszone in die Destillationszone zurückgeführt wird.

Das Verfahren kann sich auch dadurch auszeichnen, daß ein mit Feststoffpartikeln, wie z.B. Ruß, beladenes Gasgemisch eingesetzt wird, wie es bei der nicht katalytischen thermischen Spaltung von 1,2-Dichlorethan zu Vinylchlorid üblicherweise mit einer Temperatur von 480 bis 540 °C und einem Druck von 0,5 MPa bis 3,0 MPa anfällt und ggf. in einer Wärmerückgewinnung auf eine Temperatur von 180 bis 280 °C gekühlt wird. Im Fall einer katalytischen Spaltung von 1,2-Dichlorethan zu Vinylchlorid kann das eingesetzte Gasgemisch auch bei niedrigeren Temperaturen bis zu 85° C und bei niedrigerem Druck bis 0,1 MPa vorliegen.

Das Verfahren ist im Fall einer Feststoffbeladung des eingesetzten Gasgemisches dadurch gekennzeichnet, daß vor den in Anspruch 1 beschriebenen Trennvorichtungen (Verstärkungszone, Destillationszone und Abtriebszone) eine Quenchzone zur Auswaschung der Feststoffpartikel und eine Quenchsumpfaufarbeitung zur Rückgewinnung von EDC, VCM und HCL vorzusehen sind.

Die Quenchzone ist dadurch gekennzeichnet, daß ein Teilstrom des Sumpfabzuges der Verstärkungszone nach Kühlung als Quenchflüssigkeit eingesetzt wird.

Die Quenchzone ist ferner dadurch gekennzeichnet, daß

- erstens 1 bis 20 Gew.-% bezogen auf die Menge an eingesetztem Gasgemisch im Sumpf der Quenchzone flüssig als feststoffbeladener Sumpfabzug anfallen und in der Quenchsumpfaufarbeitung weiter bearbeitet werden,
- zweitens die verbleibende Menge (eingesetztes Gasgemisch plus Quenchflüssigkeit abzüglich Sumpfabzug) am Kopf der Quenchzone gasförmig als gereinigtes Quenchgas anfällt und entweder direkt oder über eine Kompressionseinheit gemäß Anspruch 2 in die Verstärkungszone geleitet wird,
- drittens die Quenchzone apparativ sowohl als eigenständiger Apparat als auch in Form eines in die Verstärkungszone integrierten Bauteils ausgeführt werden kann und
- viertens die Quenchzone apparativ als eigenständiger Apparat ausgeführt wird, falls eine Kompressionseinheit gemäß Anspruch 6 eingesetzt wird.

Die Quenchsumpfaufarbeitung ist dadurch gekennzeichnet, daß der feststoffbeladene Sumpfabzug durch abgestufte Entspannung und Verdampfung soweit eingedickt wird, daß der erhaltene Reststrom noch pumpfähig bleibt, wobei im Zuge der Ausdampfung Stoffströme erhalten werden, die in der Hauptsache EDC sowie auch HCL und VCM enthalten, und zur Rückgewinnung dieser Wertstoffe in die Abtriebszone geleitet werden.

Die Quenchsumpfaufarbeitung ist ferner dadurch gekennzeichnet, daß

- erstens in der ersten Stufe auf einen Druck entspannt wird, der etwas über dem Druck der Abtriebszone liegt,
- zweitens durch Wärmezufuhr in die erste Stufe ein Gasstrom gewonnen wird, der direkt in die Abtriebszone geleitet wird,
- drittens die verbleibende Flüssigkeit der ersten Stufe in der zweiten Stufe auf fast atmosphärischen Druck entspannt wird und
- viertens durch Wärmezufuhr in die zweite Stufe ein Gasstrom gewonnen wird, der nach Totalkondensation mittels einer Pumpe auf den Druck der Abtriebszone gebracht und in diese eingeleitet wird.

Vorteilhaft ist es darüber hinaus auch, daß das in die Quenchzone eingesetzte Gasgemisch mit einem niedrigeren Druck ausgestattet ist, als dem in der Abtriebszone vorliegenden Druck, und daß anstelle der Entspannung in der ersten Stufe der Quenchsumpfaufarbeitung der Quenchsumpf mit einer Pumpe auf einen etwas höheren Druck als den in der Abtriebszone vorliegenden Druck gebracht wird und/oder daß das eingesetzte Gasgemisch aus einem EDC-Spalt-Verfahren stammt, bei welchem mit externer EDC-Verdampfung gearbeitet wird und ein EDC-Abschlämmstrom anfällt, wobei der EDC-Abschlämmstrom in die Quenchsumpfaufarbeitung einbezogen wird und dabei

- entweder in den Quenchsumpf eingeleitet wird
- oder in der ersten Stufe der Quenchsumpfaufarbeitung die externe Wärmezufuhr mittels Einspeisung unter Entspannungsverdampfung ersetzt.

Das Verfahren der Erfindung wird nachstehend anhand der beigefügten Zeichnungen näher erläutert. Diese zeigen in:

Fig. 1     in einem Block-Fließbild die Verschaltung der Trenneinrichtungen des bisherigen Verfahrens der Spaltgasaufarbeitung,

Fig. 2     in einem Block-Fließbild die Verschaltung der bei der erfindungsgemäßen Spaltgasaufarbeitung zusammenwirkenden Stofftrennzonen und in

Fig. 3     ein Verfahrensfließbild, in dem ein Ausführungsbeispiel von den vielen anderen Ausführungsmöglichkeiten der apparativen Gestaltung der vorliegenden Erfindung dargestellt ist.

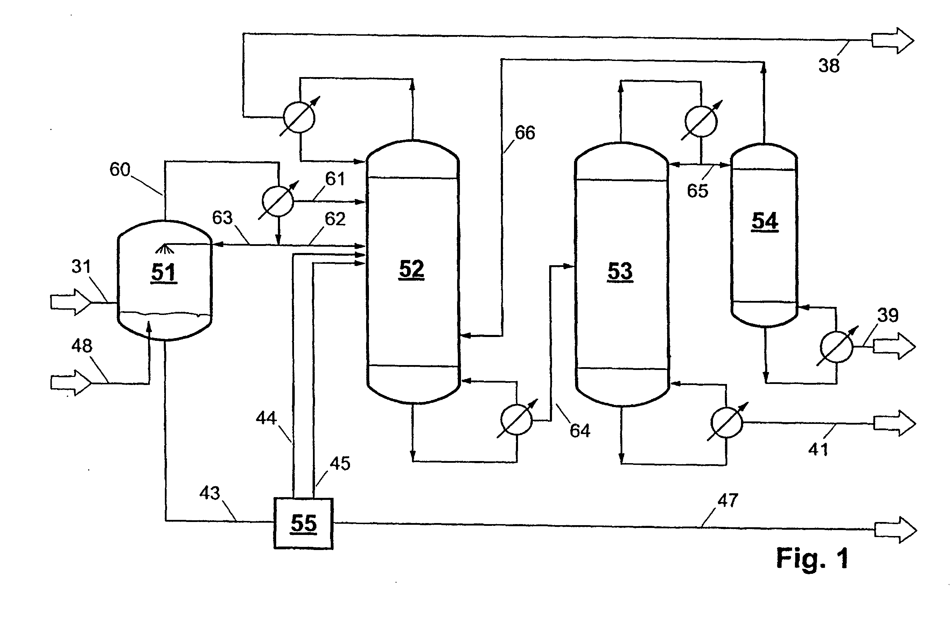

In Fig. 1 ist in einem vereinfachten Block-Fließbild das Quenchsystem 51, die HCL-Kolonne 52, die VCM-Kolonne 53, der VCM-Stripper 54 und die Quenchsumpf-Aufarbeitung 55 des bisherigen Verfahrens dargestellt.

Das eingesetzte, durch nichtkatalytische Pyrolyse von 1,2-Dichlorethan (EDC) erzeugtes Spaltgas 31 besteht aus den Hauptkomponenten HCL, VCM und nicht umgesetztem EDC. Es wird mit Temperaturen von 180 bis 540 °C, bevorzugt 230 bis 280 °C, und mit einem Druck von 0,5 MPa bis 3 MPa, bevorzugt mit einem Druck von ungefähr 2 MPa bereitgestellt. Das eingesetzte Spaltgas 31 stammt bevorzugt aus einem Verfahren nach EP-0 276 775-B1.

In dem Quenchsystem 51 wird das Spaltgas mit der Quenchflüssigkeit 63 gekühlt und von Feststoffen befreit. Die Quenchflüssigkeit 63 besteht aus einem Teilstrom des durch Kühlung des Quenchkopfdampfes 60 erhaltenen Spaltgaskondensates. In dem Verfahren entsprechend Publikation der Uhde GmbH wird weiterhin ein in der EDC-Verdampfung nach EP-0 276 775-B1 anfallender Abschlämmstrom 48 in den Sumpf des Quenchsystems 51 eingespeist. Der feststoffbeladene Quenchsumpfabzug 43 wird in der Quenchsumpfaufarbeitung 55 weiter eingedickt. Der weitgehend kondensierte Quenchkopfdampf 60 wird als Flüssigstrom 62, der aus dem Spaltgaskondensat nach Abtrennung der Quenchflüssigkeit 63 gebildet wird, und als Gasstrom 61 in die HCL-Kolonne 52 geleitet, zusammen mit den bei der Quenchsumpfaufarbeitung 55 zurückgewonnenen und vorwiegend EDC enthaltenden Produktströmen 44 und 45.

Der Feststoffe, wie Koks, und zwecks Pumpfähigkeit noch ausreichend Flüssigkeit, vorwiegend EDC, enthaltende Reststrom 47 der Quenchsumpfaufarbeitung 55 wird einer gesonderten weiteren Verarbeitung zugeführt.

Am Kopf der HCL-Kolonne 52 wird der gasförmige Produktstrom 38 abgezogen, der aus weitgehend reinem HCL-Gas besteht. Zur Erzeugung von Rücklauf für die HCL-Kolonne 52 muß Kälteenergie eingesetzt werden. Bevorzugt wird die HCL-Kolonne am Kopf mit 1,3 MPa absolut und -24° C betrieben. Um aus wirtschaftlichen Gründen den Verbrauch an teurer Kälteenergie zu beschränken, muß der Zulauf weitgehend kondensiert vorliegen. Die dem System gemäß Fig. 1 auf diese Weise entzogene Wärme muß in dem Sumpfumlaufverdampfer der HCL-Kolonne 52 wieder eingebracht werden.

Der aus den Hauptkomponenten EDC und VCM bestehende Sumpfabzug 64 der HCL-Kolonne 52 wird in die VCM-Kolonne 53 geleitet und dort in das flüssige Kopfprodukt 65, bestehend aus EDC-freiem VCM, und das Sumpfprodukt 41, in der Hauptsache aus EDC bestehend, aufgetrennt. Der Produktstrom 41 wird gesondert aufgearbeitet und das rückgewonnene EDC wird wieder in die Pyrolyse eingebracht.

Um aus wirtschaftlichen Gründen den Rücklauf für die VCM-Kolonne mit Kühlwasser erzeugen zu können, wird die Kolonne mit einem Kopfdruck von 0,6 bis 0,7 MPa absolut betrieben. Dies führt zwangsläufig zu Sumpftemperaturen von ungefähr 160° C, bei denen EDC bereits spürbar in HCL und VCM gespalten wird. Der überwiegende Teil. des neugebildeten HCL findet sich in dem Kopfprodukt 65 der VCM-Kolonne 53 wieder.

Zur Abtrennung des HCL wird das Kopfprodukt 65 der VCM-Kolonne 53 in dem VCM-Stripper 54 einer Feinreinigung unterzogen. Im Sumpf des VCM-Strippers 54 wird hochreines VCM in dem als Hauptprodukt bewerteten Produktstrom 39 abgezogen. Der Kopfdampf 66 des VCM-Strippers 54 besteht in der Hauptsache aus VCM und enthält das gesamte im Zustrom 65 zum VCM-Stripper 54 eingetragene HCL.

Der Kopfdampf 66 des VCM-Strippers 54 wird zu der HCL-Kolonne 52 zurückgeführt.

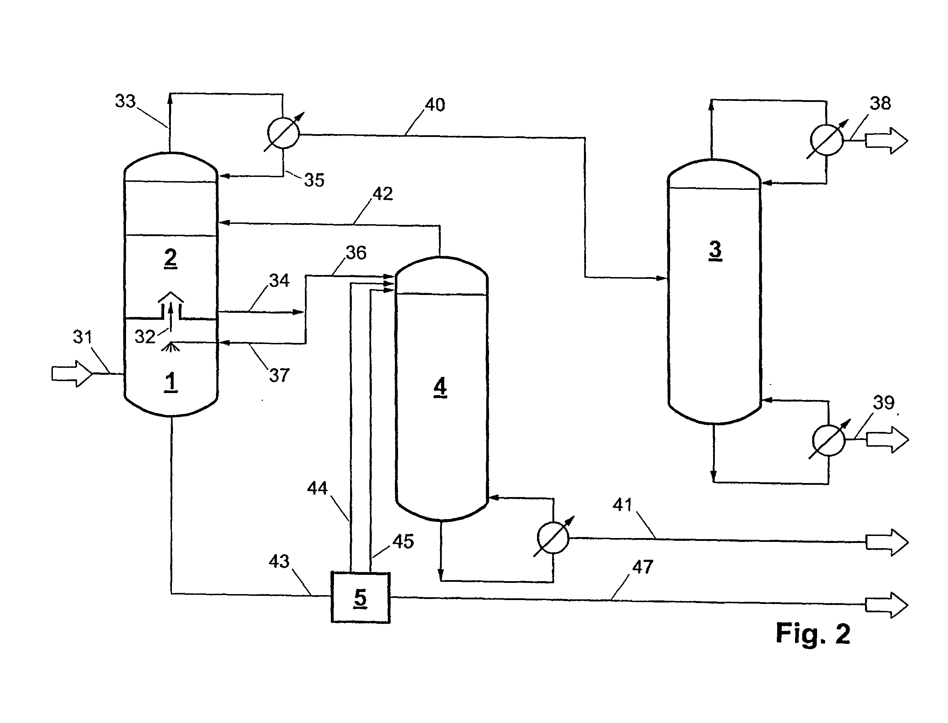

In Fig. 2 ist in einem vereinfachten Block-Fließbild die Quenchzone 1, die Verstärkungszone 2, die Destillationszone 3, die Abtriebszone 4 und die Quenchsumpfaufarbeitung 5 des erfindungsgemäßen Verfahrens dargestellt.

Im Vergleich zu dem in Fig. 1 dargestellten bisherigen Verfahren sind das eingesetzte Spaltgas 31 und dessen Auftrennung in die Produktströme 38, 39, 41 und 47 unverändert.

Das Spaltgas 31 besteht, wie bisher, aus den Hauptkomponenten HCL, VCM und nicht umgesetztem EDC und wird, wie bisher, in weitgehend reines HCL-Gas (Produktstrom 38), in das als Hauptprodukt bewertete hochreine VCM (Produktstrom 39), in den in der Hauptsache aus nicht umgesetztem EDC bestehenden Produktstrom 41 und in den feststoffhaltigen, weitgehend eingedickten Produktstrom 47 aufgetrennt.

Erfindungsgemäß neu ist die veränderte Anordnung der Aufarbeitungsvorrichtungen und die daraus resultierende erhebliche Reduzierung des Energieverbrauches.

Das Spaltgas 31 wird in der Quenchzone 1 nicht mehr, wie bisher, mit Spaltgaskondensat, sondern mit einem Teilstrom 37 des Sumpfabzuges der Verstärkungszone 2 gewaschen. Die Quenchflüssigkeit 37 des erfindungsgemäßen Verfahrens unterscheidet sich von der Quenchflüssigkeit 63 des bisherigen Verfahrens durch einen erheblichen höheren Gehalt an EDC.

Der Quenchkopfdampf 32 des erfindungsgemäßen Verfahrens wird nicht mehr kondensiert, sondern als Ersatz für einen Sumpfumlaufverdampfer direkt in die Verstärkungszone 2 geleitet.

Die Verstärkungszone 2 hat als neues Element des erfindungsgemäßen Verfahrens die Aufgabe, die Hauptkomponenten HCL und VCM frei von höher als VCM siedenden Komponenten über Kopf (Kopfprodukt 40) abzutrennen. Der Sumpfabzug 34 der Verstärkungszone 2 enthält dagegen neben HCL und VCM in der Hauptsache EDC.

Das Kopfprodukt 40 der Verstärkungszone 2 wird in die Destillationszone 3 geleitet.

Die Destillationszone 3 hat als neues Element des erfindungsgemäßen Verfahrens die Aufgabe, die Hauptkomponenten HCL und VCM in weitgehend reines HCL-Gas als Kopfprodukt 38 und in hochreines VCM als Sumpfprodukt 39 aufzutrennen.

Die Quenchsumpfaufarbeitung 5 des erfindungsgemäßen Verfahrens hat die gleiche Funktionalität wie die Quenchsumpfaufarbeitung 55 gemäß Fig. 1 des bisherigen Verfahrens.

Die rückgewonnenen Produktströme 44 und 45 aus der Quenchsumpfaufarbeitung 5 werden zusammen mit dem Zustrom 36, gebildet aus dem Sumpfabzug 34 der Verstärkungszone 2, abzüglich der erforderlichen Menge an Quenchflüssigkeit 37 zur Quenchzone 1, am Kopf der Abtriebszone 4 eingeleitet.

Die Abtriebszone 4 hat als neues Element des erfindungsgemäßen Verfahrens die Aufgabe, im Sumpf nicht umgesetztes EDC zusammen mit höher als VCM siedenden Nebenkomponenten im Produktstrom 41 abzutrennen.

Der Kopfdampf 42 der Abtriebszone 4 enthält alle Hauptkomponenten (HCL, VCM und EDC) und wird zur weiteren Abtrennung in die Verstärkungszone 2 zurückgeführt.

In der Abtriebszone 4 kommt es aufgrund der erforderlichen Temperaturen, wie in der VCM-Kolonne 53 gemäß Fig. 1 des bisherigen Verfahrens, zu einer partiellen Zersetzung des EDC zu HCL und VCM.

Aufgrund der gemäß Fig. 2 erfindungsgemäß veränderten Stoffstromzuführung mit Rückführung des Stromes 42 in die Verstärkungszone 2, erfordert die unvermeidbare thermische Instabilität des EDC keinen zusätzlichen Aufwand in einer Feinreinigungszone mehr, sondern ist vielmehr im Hinblick auf eine Steigerung der Ausbeute an VCM vorteilhaft.

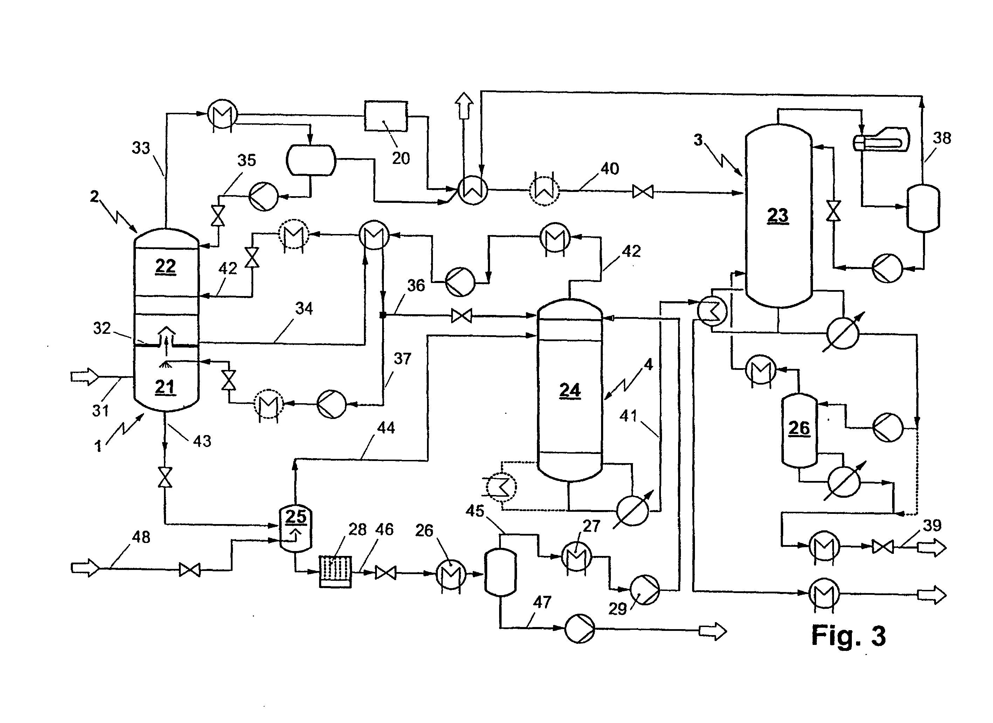

In Fig. 3 ist in einem ausführlichen Verfahrensfließbild eine bevorzugte Ausgestaltung des erfindungsgemäßen Verfahrens dargestellt. Der feststoffbeladene Spaltgaseinsatz 31 wird in den unteren Teil einer Quenchvorrichtung 21 geleitet. Die Quenchvorrichtung 21 kann als ein vertikal aufgestellter Behälter ausgeführt sein, eine bevorzugte Ausführung besteht jedoch aus dem unteren, von Einbauten freien Raum einer Destillationskolonne 22, wobei der freie Raum unterhalb der Destillationseinbauten, wie Böden oder Pakkungen, angeordnet ist. Im oberen Teil des freien Raumes der Quenchvorrichtung 21 befindet sich eine Zuführung für die Quenchflüssigkeit. Die Zuführung geht im Inneren des freien Raumes in eine geeignete Verteilereinrichtung über, mit der die gleichmäßige Beaufschlagung des gesamten freien Querschnittes geschieht, bevorzugt wird dabei ein System aus einem oder mehreren Düsenkränzen benutzt. Der Druck in der Quenchvorrichtung 21 ist durch den Druck des eintretenden Spaltgaseinsatzes 31 bestimmt.

Die Menge an Quenchflüssigkeit ist so bemessen, daß eine vollständige Auswaschung und Rückhaltung der im zuströmenden Spaltgas 31 enthaltenen Feststoffpartikel erreicht wird. Im Boden der Quenchvorrichtung 21 befindet sich ein Stutzen für die Ableitung der mit den Feststoffpartikeln beladenen Quenchflüssigkeit. Der die Quenchzone 1 oben abschließende Teil der Quenchvorrichtung 21 ist bei einer separaten Behälterlösung als ein Deckel mit Stutzen, bevorzugt jedoch bei Integration der Quenchzone 1 in eine Destillationskolonne 23, als ein Fangboden mit Gasdurchlaß für die Ableitung des von Feststoffen befreiten gasförmigen Quenchgasstromes 32 ausgeführt. Mittels dieses Durchlasses wird das Quenchgas 32 aus der Quenchzone 1 in die Verstärkungszone 2 direkt übergeleitet.

In der Verstärkungszone 2 fällt am Kopf der Kolonne 22 das überwiegend aus dem Quenchgas 32 heraus abgetrennte HCL und VCM zusammen an. Die Verstärkungszone 2 benötigt keinerlei von außen einzukoppelnde Beheizungswärme. Die Beheizung der Verstärkungszone 2 geschieht autotherm, indem für die aufzubringende Stofftrennarbeit der Wärmeinhalt des Quenchgases 32 genutzt wird, welches unten in die Verstärkungszone 2 unterhalb des untersten Teiles der Destillationseinbauten, bestehend aus handelsüblichen Böden oder Packungen, eingeleitet wird. Der Druck des von Feststoffen befreiten Quenchgases 32 bestimmt maßgeblich den Druck in der Verstärkungszone 2.

Besonders wirtschaftlich wegen des Einsatzes von ausschließlich Kühlwasser für die Rücklaufterzeugung ist ein Druck von mindestens 1,9 MPa in der Verstärkungszone 2. Dies ist gegeben, wenn das Spaltgas 31 bevorzugt aus dem Verfahren nach EP 0 276 775 Bl stammt. Falls das Spaltgas 31 aus anderen Verfahren mit niedrigerem Druck, besonders aus einer katalytischen Pyrolyse, stammt, ist eine wirtschaftliche Betrachtung maßgebend, ob die Rücklaufterzeugung bei niedrigerem Druck mittels Kälteeinsatz erfolgt, oder ob bei einer erfindungsgemäß alternativen Ausführung von Quenchvorrichtung 21 und Verstärkungszone 22 in Form von getrennten Apparaten eine Kompressionsstufe für das Quenchgas 32 zwischengeschaltet wird.

Der Rücklauf der Verstärkungszone 2 ist so bemessen, daß alle höher als VCM siedenden Komponenten zurückgehalten werden. Als Kühlmedium für einen oder mehrere Kopfkondensatoren kommt entsprechend den Druckverhältnissen Kälte, Kühlsole und/oder bevorzugt Kühlwasser zum Einsatz. Der Kopfdampf 33 der Verstärkungszone 2 wird bevorzugt nur teilweise kondensiert, damit in der verbleibenden Gasphase durch eine geeignete Trocknungsvorrichtung 20, bevorzugt gemäß Patentschrift DE-43 42 042-A1, eingeschlepptes Wasser abgetrennt werden kann. Der Sumpf 34 der Verstärkungszone 2, im wesentlichen bestehend aus EDC und darin gelöstem HCL und VCM, wird bei der bevorzugten apparativen Ausführung der Verstärkungszone 2 als Aufsatz auf die Quenchvorrichtung 21 mittels Fangboden, ansonsten aber über einen Bodenstutzen aus der Verstärkungszone 2, abgeführt.

Die in dem Sumpf 34 der Verstärkungszone 2 gelösten Mengen an HCL und VCM werden in dem erfindungsgemäßen Verfahren zurückgewonnen und in die Verstärkungszone 2 zurückgeführt. Das Verfahren sieht vor, daß der Rückstrom 42 sowohl gasförmig, als auch bevorzugt flüssig vorliegen kann. Ein gasförmiger Rückstrom 42 wird unmittelbar unter den Einbauten in die Verstärkungszone 2 eingeleitet. Im bevorzugten Fall eines flüssigen Rückstromes 42 enthält die als Verstärkungszone 2 wirkende Trennkolonne 22 eine zusätzliche Vermischungszone unter dem Einlaufstutzen für den Rückstrom 42.

Der aus der Verstärkungszone 2 abgeleitete Sumpf 34 wird gekühlt und danach in zwei Teilströme aufgetrennt.

Der eine Teilstrom des gekühlten Sumpfes 34 bildet den Vorlauf 36 für die Abtriebszone 4 und wird in die weiter unten beschriebene Abtriebszone 4 übergeleitet.

Der andere Teilstrom des gekühlten Sumpfes 34 bildet die Quenchflüssigkeit, die als EDC-angereichertes Spaltgaskondensat 37 in der Quenchzone 1 oben versprüht wird und mit dem als Kühl- und Waschmittel das Spaltgas 31 in der Quenchzone 1 behandelt wird. Ein weiterer wassergekühlter Wärmetauscher wird nur für das Anfahren der Anlage und im Störfall für eine vorübergehende zusätzliche Kühlung der Quenchflüssigkeit 37 genutzt.

Der Kopfdampf 33 der Verstärkungszone 2 mit den Hauptkomponenten HCL und VCM wird erfindungsgemäß nach Totalkondensation oder bevorzugt nach Teilkondensation und nach Abtrennung des Rücklaufes 35 als verbleibender Zustrom 40 einer Destillationszone 3 zugeführt, die als übliche Destillationskolonne 23 mit Rektifikationszone oberhalb und Strippzone unterhalb des Zulaufes ausgeführt ist. Die Destillationseinbauten bestehen aus handelsüblichen Böden oder Pakkungen. Am Kopf der Kolonne 23 wird weitgehend reines HCL 38 und im Sumpf wird hochreines VCM 39 nach optionalem Durchlauf durch die Feinreinigungszone 26 abgezogen. Der Betrieb der Feinreinigungszone 26 ist im Gegensatz zu dem bisherigen Verfahren (VCM-Stripper 54 in Fig. 1) nicht dauernd erforderlich, sondern stellt eine Sicherheitseinrichtung dar, mit welcher nur im Fall eines nicht optimalen Arbeitens der Destillationszone 3 ein möglicherweise drohender HCL-Durchbruch in den VCM-Produktstrom 39 vermieden wird. Im Fall des optimalen Arbeitens der Destillationszone 3 wird die Feinreinigungszone 26 überbrückt, wie in Fig. 3 mittels gestrichelter Linie dargestellt. Der Kolonnensumpf wird mit einem oder mehreren Umlaufverdampfern beheizt, von denen ein Umlaufverdampfer bevorzugt mit Dampf zur Einstellung der VCM-Produktqualität beheizt wird. Weiterhin wird bevorzugt, einen oder mehrere Umlaufverdampfer zusätzlich mit geeigneten Produktströmen zu beheizen. Der Druck in dieser Destillationszone 3 wird von dem zur Rückflußerzeugung eingesetzten Kühlmittel bestimmt, bevorzugt werden Kältemittel, wie z.B. verdampfendes Propylen. Aus Gründen des wirtschaftlichen Einsatzes von Kältemittel kann es sinnvoll sein, den Zulauf 40 vorzukühlen, bevorzugt im Gegenstrom mit dem Kopfprodukt, aber auch mit einer zusätzlichen Kühlvorrichtung. Der Druck der Destillationszone 3 liegt bevorzugt unter dem Druck der Verstärkungszone 2.

Das erfindungsgemäße Verfahren kann auch solches durch Pyrolyse von 1,2-Dichlorethan (EDC) erzeugtes Spaltgas 31 aufarbeiten, das mit relativ niedrigem Druck als Einsatz übergeben wird. Dadurch nimmt der Druck in der Verstärkungszone 2 ebenfalls relativ niedrige Werte an. In diesem Fall kann der Zulauf 40 zur Destillationszone 3 durch ein geeignetes Kühlmittel total kondensiert und mit einer Pumpe auf einen höheren Druck gebracht werden, um eine wirtschaftliche Trennung von HCL und VCM zu erreichen.

Der eine gekühlte Teilstrom des Sumpfes 34 aus der Verstärkungszone 2 bildet den Vorlauf 36 für die Abtriebszone 4. Der Vorlauf 36 wird in die Abtriebszone 4 oben, und zwar oberhalb der Destillationseinbauten, eingeleitet. In diesem oberen Raum der Abtriebszone 4 ist eine Vermischungszone für die Aufnahme weiterer Stoffströme des erfindungsgemäßen Verfahrens angeordnet. Diese weiteren Stoffströme sind aus anderen Aufarbeitungen rückgeführt, weil sie neben EDC noch HCL und VCM enthalten. Durch ihre Rückführung in die Abtriebszone 4 gehen die betreffenden Mengen der aus dem Spaltgas 31 stammenden Hauptkomponenten nicht verloren.

Der untere Teilraum der Abtriebszone 4 ist mit den Destillationseinbauten ausgestattet. In diesem unteren Teilraum der Abtriebszone 4 erfolgt die Abtrennung des im Vorlauf 36 für die Abtriebszone 4 enthaltenen, nicht umgesetzten EDC. Das nicht umgesetzte EDC wird weitgehend frei von HCL und VCM als Sumpfprodukt 41 der Abtriebszone 4 gewonnen.

Die Destillationseinbauten in der Abtriebskolonne 24 bestehen aus handelsüblichen Böden oder Packungen. Der Kolonnensumpf wird mit einem oder mehreren Umlaufverdampfern beheizt, von denen ein Umlaufverdampfer bevorzugt mit Dampf zur Einstellung eines möglichst geringen VCM-Verlustes im Sumpfprodukt beheizt wird. Weiterhin wird bevorzugt, einen oder mehrere Umlaufverdampfer zusätzlich zwecks Energieeinsparung mit geeigneten Produktströmen zu beheizen, z.B. gemäß Patentschrift DE-41 31 576-A1.

Das gasförmige Kopfprodukt 42 der Abtriebszone 4 enthält rückgewonnenes HCL und VCM sowie nicht umgesetztes EDC. Das Kopfprodukt 42 der Abtriebszone 4 wird in die Verstärkungszone 2 zurückgeführt. Der in der Abtriebszone 4 eingestellte Druck stellt eine wirtschaftliche Betriebsweise sicher. Es wird damit erreicht, daß erstens die Energiekosten minimiert werden und daß zweitens das Kopfprodukt 42 auf einem Temperaturniveau größer 100° C gehalten wird. Bei einer derart angehoben Kopftemperatur der Abtriebszone 4 wird die Ausbeute an VCM gesteigert, weil eine partielle thermische Zersetzung des EDC zu VCM und HCL dann ohne weiteres Zutun von selbst geschieht.

Der Druck in der Abtriebszone 4 wird bevorzugt auf niedrige Werte gegenüber der Verstärkungszone 2 eingestellt, sofern das eingesetzte Spaltgas 31 nach der in EP-0 276 775-B1 beschriebenen Methode hergestellt wird und in dem dabei bevorzugten Druckbereich von 1,6 bis 2,6 MPa vorliegt. Ein Weg zur Druckerhöhung des gasförmig aus der Abtriebszone 4 abgezogenen Kopfproduktes 42 besteht in der Totalkondensation des Kopfproduktes 42, seine anschließende Druckerhöhung durch eine Pumpe und sein daraufhin erfolgendes Aufheizen im Gegenstrom mit dem Sumpfprodukt 34 der Verstärkungszone 2. Bei einer alternativ beschreitbaren Variante wird das gasförmige Kopfprodukt 42 der Abtriebszone 4 mittels einer Kompressionseinheit auf einen Druck gebracht, der die Rückführung des Kopfproduktes 42 der Abtriebszone 4 in die druckmäßig höher betriebene Verstärkungszone 2 gestattet. Ein weiterer dampfbeheizter Wärmetauscher wird nur zusätzlich für das Anfahren der Anlage und im Störfall für eine vorübergehende Aufheizung genutzt.

Der Druck in der Abtriebszone 4 kann aber auch auf gegenüber dem Druck in der Verstärkungszone 2 überhöhte Werte eingestellt werden. In diesem Fall wird der aus der Verstärkungszone 2 in die Abtriebszone 4 übergeleitete Vorlauf 36 der Abtriebszone 4 mit einer Pumpe auf den in der Abtriebszone 4 herrschenden höheren Druck gebracht.

Das Sumpfprodukt der Quenchzone 1, der Sumpfablauf 43, wird in dem erfindungsgemäßen Verfahren mit dem Ziel der Rückgewinnung von HCL und VCM aufgearbeitet. Dies geschieht mittels der einander nachgeordneten Verfahrensschritte Filtration, stufenweise Entspannung und partielle Verdampfung.

Wird das Verfahren gemäß vorliegender Erfindung zusammen mit dem Verfahren gemäß Patentschrift EP-0 276 775-B1 genutzt, so fällt bei der Variante mit externer EDC-Verdampfung ein im wesentlichen aus reinem EDC bestehender flüssiger Abschlämmstrom 48 in einer Verdampfungszone an, deren Temperatur unter dem bzw. fast am Siedepunkt des reinen EDC's liegt. Diese Verdampfungszone arbeitet bei einem höheren Druck als dem in der Quenchzone 1 eingestellten Druck.

Dieser Abschlämmstrom 48, mit dem im zu verdampfenden EDC die Menge der Precursoren bzw. Keime für die bei der pyrolytischen EDC-Spaltung eintretende Rußbildung vermindert werden soll, wird in die Quenchsumpfaufarbeitung einbezogen. Der Abschlämmstrom 48 wird in dem bisherigen Verfahren gemäß Fig. 1 in die Quenchvorrichtung 51 unterhalb des Flüssigkeitsniveaus des Quenchsumpfes eingespeist, um in der Hauptsache gelöstes HCL und VCM zu verdampfen.

Weiterhin wird in dem bisherigen, in Fig. 1 dargestellten Verfahren, der Sumpfabzug 43 der Quenche 51 filtriert, auf einen etwas höheren Druck als den der HCL-Kolonne 52 entspannt und mit einem dampfbeheizten Wärmeaustauscher aufgeheizt, um einen HCL, VCM und EDC enthaltenden Gasstrom 44 zu gewinnen, der zwecks Rückgewinnung der Produkte zur HCL-Kolonne 52 geleitet wird.

Erfindungsgemäß wird in dem hier angegebenen Verfahren eine andere Form der Einspeisung des Abschlämmstromes 48 bevorzugt, die derart einfach gestaltet ist, daß durch den Einsatz lediglich einer einstufigen Entspannungsverdampfung des Abschlämmstromes 48 in einen Behälter 25 hinein der erste Wärmeaustauscher des bisherigen Verfahrens einschließlich des damit verbundenen Heizenergieverbrauches eingespart wird. Die erfindungsgemäße Gestaltung sieht vor, daß der Sumpfablauf 43 aus der Quenchzone 1 direkt in einen Behälter 25 entspannt wird und der nach Entspannung überwiegend gasförmig anfallende Abschlämmstrom 48 mittels einer geeigneten Vorrichtung in die Flüssigphase des Behälters 25 geleitet wird.

Der verdampfte Teil des Sumpfablaufes 43 wird als erster Gasstrom 44 vom Sumpfablauf 43 abgetrennt und unterhalb der Vermischungszone in die Abtriebszone 4 geleitet. Der verbleibende erste Flüssigkeitsstrom 46 wird anschließend in der Filtrationseinheit 28 von abtrennbaren Feststoffen befreit und auf einen etwas über dem Atmosphärendruck liegenden Druck entspannt. Nachdem dem ersten Flüssigkeitsstrom 46 weitere Wärme in dem Wärmeaustauscher 26 zugeführt und dadurch ein weiterer Teil des ursprünglichen Sumpfablaufes 43 verdampft wurde, wird ein zweiter Gasstrom 45 abgetrennt. Der verbleibende zweite Flüssigkeitsstrom 47 ist weitgehend von HCL und VCM befreit und wird einer nicht zum erfindungsgemäßen Verfahren gehörenden Aufarbeitung zugeführt. Der zweite Gasstrom 45 wird mit Kühlwasser im Wärmeaustauscher 27 total kondensiert, mit einer Pumpe 29 auf den Druck der Abtriebszone 4 gebracht und in diese oberhalb der Destillationseinbauten eingeleitet.

Das erfindungsgemäße Verfahren ist in der Lage, ein technisch sowohl mittels katalysatorfreier als auch mittels katalytischer Pyrolyse hergestelltes Spaltgas 31 aufzuarbeiten. Das mittels Pyrolyse erzeugte heiße Spaltgas 31 besteht aus seinen Hauptkomponenten HCL, VCM und nicht umgesetztem EDC. Im Fall eines Verfahrens der thermischen Spaltung von 1,2-Dichlorethan, bei dem es gelingt, die Bildung von Feststoffen, wie Ruß und/oder Koks, zu vermeiden, entfallen bei dem in Fig. 2 als Block-Fließbild dargestellten erfindungsgemäßen Verfahren die Anlagenteile 1 (Quenchzone) und 5 (Quenchsumpfaufarbeitung). Im Fall eines feststofffrei arbeitenden, atmosphärisch-katalytischen Verfahrens zur thermischen Spaltung von EDC ist das erfindungsgemäße Verfahren mit einer Kompressionsstufe vor dem Eintritt des Spaltgases 31 in die Verstärkungszone 2 zu versehen.

Die wirtschaftlichen Vorteile, die bei der Nutzung des erfindungsgemäßen Verfahrens eintreten, bestehen darin, daß bei ungefähr gleichbleibendem Verbrauch an Kälte- und Pumpenenergie, sowie ungefähr gleichbleibenden Investitionskosten der Verbrauch an Dampf und Kühlwasser erheblich reduziert wird.

In der bevorzugten Anwendung gemäß Fig. 3 ergeben sich für ein Spaltgas und einen EDC-Abschlämmungsstrom aus einem Verfahren nach EP-0 276 775-B1 folgende Verbrauchswerte im Vergleich mit dem bisherigen Verfahren gemäß Fig. 1:

- ein konstanter Verbrauch an Kälteenergie,
- eine Erhöhung der Netto-Pumpenleistung um 5 %,
- ein Reduzierung des Verbrauchs an Mitteldruckdampf um 67 %,
- eine Reduzierung des Verbrauchs an Niederdruckdampf um 80 % und
- eine Reduzierung des Verbrauchs an Kühlwasser um 36 %.

Bezogen auf eine 100000 jato VCM-Anlage mit einer Betriebszeit von 8000 Stunden pro Jahr bedeutet dies jährliche Einsparungen von 26700 t Mitteldruckdampf und von 20280 t Niederdruckdampf.

**Patentansprüche**

1. Verfahren zur Aufbereitung eines durch Pyrolyse von 1,2-Dichlorethan (EDC) entstandenen Spaltgases, bei dem das Spaltgas in seine Hauptkomponenten Chlorwasserstoff (HCl), Vinylchlorid (VCM) und nicht umgesetztes 1,2-Dichlorethan (EDC) zerlegt wird und diese in weitgehend reiner Form anfallen, wobei im ersten Schritt der Aufbereitung des Spaltgases von diesem die Feststoffe in einer Quenchzone abgetrennt werden und das Verfahren **dadurch gekennzeichnet ist, daß**:

   - in einer der Quenchzone (1) folgenden Verstärkungszone (2) das gesamte HCl und VCM als Kopfprodukt abgezogen und einer Destillationszone (3) zur Trennung zugeführt wird,

   - ein Teil des Sumpfproduktes der Verstärkungszone (2) einer Abtriebszone (4) zugeführt (36) wird, der andere Teil des Sumpfproduktes der Verstärkungszone (2) als ein mit EDC angereichertes, von Feststoffen freies Spaltgaskondensat als Quenchflüssigkeit (37) in der Quenchzone (1) durch Rückführung eingesetzt wird, und daß

   - das Kopfprodukt der Abtriebszone (4) vollständig in die Verstärkungszone (2) zurückgeführt wird.

2. Verfahren nach Anspruch 1, bei dem Spaltgas aufgearbeitet wird, welches bei der nicht katalytischen thermischen Spaltung von 1,2-Dichlorethan zu Vinylchlorid mit einer Temperatur von 480 bis 540 °C und einem Druck von 0,5 MPa bis 3,0 MPa anfällt und ggf. in einer Wärmerückgewinnung auf eine Temperatur von 180 bis 280 °C gekühlt und mit dieser Temperatur in die Quenchzone geleitet wird, in der es mit Spaltgaskondensat gekühlt und gewaschen wird,

   wobei das Verfahren **dadurch gekennzeichnet ist, daß** man:

   - 80 bis 99 Gew.-% der abgekühlten Spaltgase gasförmig als Brüden am Kopf der Quenchzone als Quenchgas entnimmt,

   - und 1 bis 20 Gew.-% der abgekühlten Spaltgase als Sumpfablauf der Quenchzone flüssig entnimmt,

   - das hierbei erhaltene Quenchgas unmittelbar unten in eine Verstärkungszone einleitet und das Quenchgas darin in ein Destillat und einen Sumpf auftrennt,

   - den in der Verstärkungszone anfallenden Sumpf ableitet und so das besagte, mit 1,2-Dichlorethan (EDC) angereicherte, von Feststoffen freie Spaltgaskondensat gewinnt und es in zwei Teilströme auftrennt und als den einen Teilstrom den Vorlauf für die Abtriebszone erhält und als den anderen Teilstrom jenes EDC-angereicherte, feststofffreie Spaltgaskondensat erhält, welches in die Quenchzone zurückgeleitet wird, um darin mit ihm als Kühl- und Waschmittel das aufzuarbeitende Spaltgas zu behandeln;

   - und daß man das Destillat und den Vorlauf getrennt den weiteren destillativen Aufarbeitungen zuführt

   - und den aus der Quenchzone kommenden, mit dem auszuschleusenden Feststoff beladenen Sumpfablauf in eine andere Aufarbeitung einleitet.

3. Verfahren nach Anspruch 1 oder 2, **dadurch gekennzeichnet**, **daß** die destillative Aufarbeitung des Vorlaufes in der Abtriebszone geschieht, mit dem darin gebildeten Sumpfprodukt das angereichert rückgewonnene 1,2-Dichlorethan (EDC) abgezogen wird und das darin gebildete Kopfprodukt in die Verstärkungszone zurückgeleitet wird.

4. Verfahren nach einem der voranstehenden Ansprüche, **dadurch gekennzeichnet**, **daß** die destillative Aufarbeitung des Destillates in der Destillationszone geschieht, mit dem darin gebildeten Sumpfprodukt das abgetrennte Vinylchlorid (VCM) hoch angereichert abgezogen wird und mit dem darin gebildeten Kopfprodukt der Chlorwasserstoff (HCL) angereichert rückgewonnen wird.

5. Verfahren nach Anspruch 1 oder einem der folgenden, zur destillativen Zerlegung eines durch katalytische oder nicht katalytische, unter Zufuhr von thermischer Energie erfolgten Zersetzung von 1,2-Dichlorethan (EDC) entstandenen und zuvor ggf. von Feststoffen befreiten Gasgemisches in eine weitgehend in reiner Form anfallenden Hauptkomponenten Chlorwasserstoff (HCL), Vinylchlorid (VCM) und nicht umgesetztes 1,2-Dichlorethan (EDC), wobei das Verfahren **dadurch gekennzeichnet ist, daß**

- erstens das zu trennende Gasgemisch mit Temperaturen von 85 bis 540 °C und mit einem Druck von 0,1 MPa bis 3,0 MPa vorliegen kann,
- zweitens das zu trennende Gasgemisch vor Eintritt in die erste Destillationskolonne gasförmig unter die Einbauten einer Verstärkungszone geleitet wird,
- drittens am Kopf der Verstärkungszone ein Gemisch aus HCL, VCM und leichter als VCM siedenden Komponenten abgezogen und als Zulauf zu einer Destillationszone geführt wird,
- viertens als Sumpfprodukt der Verstärkungszone ein Gemisch anfällt, welches aus EDC als Hauptkomponente und aus HCL,VCM und höher als VCM siedenden Komponenten als Nebenkomponenten besteht und welches als Teilstrom, falls Quenchflüssigkeit für eine zuvor zu erfolgende Abtrennung von Feststoffen aus dem eingesetzten Gasgemisch benötigt wird, oder als Gesamtstrom zu einer Abtriebszone geleitet wird,
- fünftens das Kopfprodukt der Verstärkungszone in der Destillationszone in hochreines VCM, welches als Sumpfprodukt der Destillationszone anfällt, und in weitgehend reines HCL, welches aus HCL und leichter als VCM siedenden Komponenten besteht und am Kopf der Destillationszone anfällt, aufgetrennt wird und
- sechstens in der Abtriebszone das aus dem Sumpf der Verstärkungszone zulaufende Gemisch aufgetrennt wird, wobei im Sumpf der Abtriebszone weitgehend reines EDC mit allen höher als VCM siedenden Komponenten und nur sehr geringem Gehalt an HCL und VCM abgezogen wird, während am Kopf der Abtriebszone ein Gemisch gewonnen wird, welches aus EDC und im Zulauf eingetragenem HCL und VCM sowie durch die thermische Instabilität von EDC neu gebildetem HCL und VCM besteht, und zwecks weiterer Aufarbeitung in die Verstärkungszone zurückgeführt wird.

6. Verfahren nach Anspruch 1 oder einem der folgenden, **dadurch gekennzeichnet, daß** ein in die Verstärkungszone eingesetztes, von Feststoffen befreites, aber mit relativ niedrigem Druck ausgestattetes Gasgemisch mit einer Kompressionseinheit auf einen Druck gebracht wird, der ein wirtschaftliches Arbeiten der Verstärkungszone sichert.

7. Verfahren nach Anspruch 1 oder einem der folgenden, **dadurch gekennzeichnet, daß** die Abtriebszone sowohl bei niedrigerem als auch bei höherem Druck als dem in der Verstärkungszone verwendeten Druck betrieben wird.

8. Verfahren nach Anspruch 1 oder einem der folgenden, **dadurch gekennzeichnet, daß** einer oder mehrere Sumpfumlaufverdampfer der Destillationszone mit dem Sumpfprodukt der Abtriebszone beheizt werden.

9. Verfahren nach Anspruch 1 oder einem der folgenden, **dadurch gekennzeichnet, daß** das Sumpfprodukt der Destillationszone über eine wahlweise zuschaltbare Feinreinigungszone geleitet wird, wobei ein hochreiner Produktstrom im Sumpf der Feinreinigungszone abgezogen wird und das Kopfprodukt der Feinreinigungszone in die Destillationszone zurückgeführt wird.

10. Verfahren nach Anspruch 1 oder einem der folgenden, **dadurch gekennzeichnet, daß** ein mit Feststoffpartikeln, wie z.B. Ruß, beladenes Gasgemisch eingesetzt wird, mit einer Temperatur von 480 bis 540 °C und einem Druck von 0,5 MPa bis 3,0 MPa anfällt und ggf. in einer Wärmerückgewinnung auf eine Temperatur von 180 bis 280 °C gekühlt wird.

11. Verfahren nach den Ansprüchen 1 bis 6, **dadurch gekennzeichnet, daß** das in die Quenchzone eingesetzte Gasgemisch mit einem niedrigeren Druck ausgestattet ist, als dem in der Abtriebszone vorliegenden Druck, und daß anstelle der Entspannung in der ersten Stufe der Quenchsumpfaufarbeitung der Quenchsumpf mit einer Pumpe auf einen etwas höheren Druck als den in der Abtriebszone vorliegenden Druck gebracht wird.

12. Verfahren nach den Ansprüchen 1 bis 7, **dadurch gekennzeichnet, daß** das eingesetzte Gasgemisch aus einem EDC-Spalt-Verfahren stammt, bei welchem mit externer EDC-Verdampfung gearbeitet wird und ein EDC-Abschlämmstrom anfällt, wobei der EDC-Abschlämmstrom in die Quenchsumpfaufarbeitung einbezogen wird und dabei

- entweder in den Quenchsumpf eingeleitet wird
- oder in der ersten Stufe der Quenchsumpfaufarbeitung die externe Wärmezufuhr mittels Einspeisung unter Entspannungsverdampfung ersetzt.

## Claims

1. A process for treating a cracking gas produced by pyrolysis of 1,2-dichloroethane (EDC), in which the cracking gas is broken down into its main components hydrogen chloride (HCl), vinyl chloride (VCM) and unreacted 1,2-dichloroethane (EDC), these being obtained substantially in pure form, the first cracking gas treatment step comprising separation of the solids from the cracking gas in a quenching zone and the process being **characterised in that**:

   - in a rectification zone (2) following the quenching zone (1), the entire HCl and VCM are drawn off as overhead product and supplied to a distillation zone (3) for separation,
   - part of the bottom product of the rectification zone (2) is fed to a stripping zone (4), the other part of the bottom product of the rectification zone (2) being used in the form of an EDC-enriched, solids-free cracking gas condensate as quenching fluid (37) in the quenching zone (1) by recycling, and **in that**
   - the overhead product of the stripping zone (4) is returned completely to the rectification zone (2).

2. A process according to claim 1, in which cracking gas is worked up which is obtained during non-catalytic thermal cracking of 1,2-dichloroethane to yield vinyl chloride at a temperature of 480 to 540 °C and a pressure of 0.5 MPa to 3.0 MPa and optionally cooled by heat recovery to a temperature of 180 to 280 °C and conveyed at this temperature into the quenching zone, in which it is cooled and washed with cracking gas condensate, wherein the process is **characterised in that**:

   - 80 to 99 wt.% of the cooled cracking gases are removed in gaseous form as vapours at the top of the quenching zone as quench gas,
   - and 1 to 20 wt.% of the cooled cracking gases are removed in liquid form as bottoms runoff from the quenching zone,
   - the quench gas obtained in this way is introduced directly into the bottom of a rectification zone and the quench gas is separated therein into a distillate and bottoms,

   - the bottoms arising in the rectification zone are drawn off and said solids-free cracking gas condensate enriched with 1,2-dichloroethane (EDC) is thus recovered and divided into two substreams and the first running for the stripping zone is obtained as the one substream and the EDC-enriched, solids-free cracking gas condensate which is recirculated into the quench zone is obtained as the other substream, in order to treat therewith as coolant and washing agent in said quench zone the cracking gas to be worked up;
   - and **in that** the distillate and the first running are fed separately to the further distillatory working-up processes
   - and the solids-charged bottoms runoff to be discharged, coming from the quench zone, is introduced to another working-up process.

3. A process according to claim 1 or claim 2, **characterised in that** distillatory working-up of the first running takes place in the stripping zone, the 1,2-dichloroethane (EDC), recovered in an enriched state, is drained off with the bottom product formed in the stripping zone and the overhead product formed therein is recycled into the rectification zone.

4. A process according to one of the preceding claims, **characterised in that** distillatory working-up of the distillate takes place in the distillation zone, the separated-off vinyl chloride (VCM) is drawn off in a highly enriched state with the bottom product formed in the distillation zone and the hydrogen chloride (HCl) is recovered in an enriched state with the overhead product formed therein.

5. A process according to claim 1 or one of the following claims, for distillatory fractionation of a gas mixture, obtained with input of thermal energy by catalytic or non-catalytic breakdown of 1/2-dichloroethane (EDC) and optionally previously freed from solids, into the main components, obtained substantially in pure form, hydrogen chloride (HCl), vinyl chloride (VCM) and unreacted 1,2-dichloroethane (EDC), the process being **characterised in that**

   - firstly, the gas mixture to be separated may be present at temperatures of 85 to 540 °C and at a pressure of 0.1 MPa to 3.0 MPa
   - secondly, prior to entry into the first distillation column, the gas mixture to be separated is passed in gaseous form beneath the internals of a rectification zone,

- thirdly, a mixture of HCl, VCM and lower boiling components than VCM is drawn off at the top of the rectification zone and fed to a distillation zone as influent,
- fourthly, a mixture is obtained as bottom product of the rectification zone, which mixture consists of EDC as main component and of HCl, VCM and higher boiling components than VCM as secondary components and which is passed to a stripping zone as a substream, if quench fluid is required for upstream separation of solids from the gas mixture used, or as a total stream,
- fifthly, the overhead product of the rectification zone is separated in the distillation zone into high purity VCM, which is obtained as the bottom product of the distillation zone, and substantially pure HCl, which consists of HCl and lower boiling components than VCM and is obtained at the top of the distillation zone, and
- sixthly, the mixture flowing in from the bottom of the rectification zone is separated in the stripping zone, wherein substantially pure EDC is drawn off in the bottom of the stripping zone with all the higher boiling components than VCM and only a very small content of HCl and VCM, while a mixture is recovered at the top of the stripping zone, which consists of EDC and of HCl and VCM introduced in the influent, together with HCl and VCM newly formed by the thermal instability of EDC, and is recycled into the rectification zone for further working up.

6. A process according to claim 1 or one of the following claims,
   **characterised in that**
   a solids-free, relatively low pressure gas mixture introduced into the rectification zone is brought with a compression unit to a pressure which ensures economic operation of the rectification zone.

7. A process according to claim 1 or one of the following claims,
   **characterised in that**
   the stripping zone is operated both at lower and higher pressures than the pressure used in the rectification zone.

8. A process according to claim 1 or one of the following claims,
   **characterised in that**
   one or more bottoms circulating evaporators of the distillation zone are heated with the bottom product of the stripping zone.

9. A process according to claim 1 or one of the following claims,
   **characterised in that**
   the bottom product of the distillation zone is passed through a fine purification zone which may optionally be connected, wherein a highly pure product stream is drawn off in the bottoms of the fine purification zone and the overhead product of the fine purification zone is recycled to the distillation zone.

10. A process according to claim 1 or one of the following claims,
    **characterised in that**
    a gas mixture charged with solids particles, such as for example carbon black, is obtained at a temperature of 480 to 540 °C and a pressure of 0.5 MPa to 3.0 MPa and optionally cooled by heat recovery to a temperature of 180 to 280 °C.

11. A process according to claims 1 to 6,
    **characterised in that**
    the gas mixture introduced into the quench zone is at a lower pressure than the pressure prevailing in the stripping zone, and **in that**, instead of the flash in the first stage of quench bottoms working-up, the quench bottoms are brought with a pump to a somewhat higher pressure than the pressure prevailing in the stripping zone.

12. A process according to claims 1 to 7,
    **characterised in that**
    the gas mixture used stems from an EDC cracking process, which uses external EDC vaporisation and in which an EDC blow-down stream is obtained, wherein the EDC blow-down stream is used in quench bottoms working-up and

- is either introduced into the quench bottoms
- or replaces the external heat supply in the first stage of quench bottoms working-up by charging accompanied by flash vaporisation.

## Revendications

1. Procédé pour retraiter un gaz de craquage formé par pyrolyse du 1,2-dichloroéthane (EDC), dans lequel le gaz de craquage est fragmenté en ses composants principaux, à savoir le gaz chlorhydrique (HCl), le chlorure de vinyle (VCM) et le 1,2-dichloroéthane (EDC) non transformé, ces composants se présentant sous une forme assez pure, dans lequel dans la première étape de retraitement du gaz de craquage les matières solides en sont séparées dans une zone de refroidissement, le procédé étant **caractérisé en ce que** :

- dans une zone de renforcement (2) qui fait suite à la zone de refroidissement (1) la totalité du HCl et du VCM est retirée en tant que produit de tête et est conduite vers une zone de distillation (3) pour la séparation,

- une partie du produit de fond de la zone de renforcement (2) est conduite (36) vers une zone de rectification (4), l'autre partie du produit de fond de la zone de concentration (2) qui constitue un condensat de gaz de craquage exempt de matières solides et enrichi en EDC est recyclée en tant que liquide de refroidissement (37) dans la zone de refroidissement (1) et **en ce que**
- le produit de tête de la zone de rectification (4) est recyclé en totalité dans la zone de concentration (2).

2. Procédé selon la revendication 1 dans lequel on retraite du gaz de craquage, qui est obtenu lors du craquage thermique non catalytique de 1,2-dichloroéthane en chlorure de vinyle à une température de 480 à 540 °C et une pression de 0,5 MPa à 3,0 MPa, ce gaz de craquage étant éventuellement refroidi à une température de 180 à 280 °C avec récupération de chaleur et est conduit à cette température dans la zone de refroidissement où il est refroidi et lavé avec un condensat de gaz de craquage, ledit procédé étant **caractérisé en ce que**

- l'on prélève en tant que gaz de refroidissement de 80 à 99 % en poids des gaz de craquage refroidis en tant que vapeur à la tête de la zone de refroidissement,
- l'on prélève de la zone de refroidissement sous forme liquide en tant qu'écoulement de fond de 1 à 20 % en poids des gaz de craquage refroidis,
- l'on introduit le gaz de refroidissement ainsi obtenu directement en bas dans une zone de concentration dans laquelle le gaz de refroidissement est séparé en un distillat et en un fond,
- l'on évacue le fond obtenu dans la zone de concentration et on obtient ainsi ledit condensat de gaz de craquage exempt de matières solides et enrichi en 1,2-dichloroéthane (EDC) et on le sépare en deux courants partiels et on obtient en tant qu'un des écoulements partiels la fraction de tête pour la zone de rectification et on obtient en tant que l'autre écoulement partiel le condensat de gaz de craquage exempt de matières solides enrichi en EDC qui est recyclé dans la zone de refroidissement pour y traiter grâce à lui, en tant qu'agent de refroidissement et de lavage, le gaz de craquage à retraiter,
- et qu'on conduit le distillat et la fraction de tête séparément aux autres retraitements par distillation,
- et qu'on introduit dans un autre retraitement l'écoulement de fond chargé des matières solides à séparer provenant de la zone de refroidissement.

3. Procédé selon la revendication 1 ou 2 **caractérisé en ce que** le retraitement par distillation de la fraction de tête a lieu dans la zone de rectification, le 1,2-dichloroéthane (EDC) récupéré de façon enrichie est enlevé avec le produit de fond qui y est formé et le produit de tête qui y est formé est recyclé dans la zone de concentration.

4. Procédé selon l'une des revendications précédentes **caractérisé en ce que** le retraitement par distillation du distillat a lieu dans la zone de distillation, **en ce que** le chlorure de vinyle (VCM) séparé est enlevé de façon fortement enrichie avec le produit de fond qui y est formé et le gaz chlorhydrique (HCl) est récupéré de façon enrichie avec le produit de tête qui y est formé.

5. Procédé selon la revendication 1 ou l'une des suivantes pour le fractionnement par distillation d'un mélange de gaz débarrassé éventuellement auparavant de matières solides, formé par décomposition catalytique ou non catalytique avec apport d'énergie thermique de 1,2-dichloroéthane, en ses composants principaux obtenus essentiellement sous forme pure, à savoir le gaz chlorhydrique (HCl), le chlorure de vinyle (VCM) et le 1,2-dichloroéthane (EDC) non transformé, ledit procédé étant **caractérisé en ce que**

- premièrement le mélange gazeux à séparer peut avoir des températures comprises de 85 à 540 °C et une pression de 0,1 MPa à 3,0 MPa,
- deuxièmement le mélange gazeux à séparer est conduit sous forme gazeuse avant l'entrée dans la première colonne de distillation sous les éléments d'une zone de concentration,
- troisièmement on retire à la tête de la zone de concentration un mélange de HCl, de VCM et de composants à température d'ébullition plus basse que celle du VCM et on le conduit vers une zone de distillation en tant qu'amenée,
- quatrièmement est obtenu en tant que produit de fond de la zone de concentration un mélange qui se compose d'EDC en tant que composant principal et de HCl, de VCM et de composants à température d'ébullition plus élevée que celle du VCM en tant que composants auxiliaires et qui est conduit à une zone d'enlèvement en tant qu'écoulement partiel, au cas où un liquide de refroidissement est nécessaire pour une séparation à effectuer préalablement de matières solides hors du mélange gazeux utilisé, ou est conduit en tant qu'écoulement total à une zone de rectification ou d'enlèvement,
- cinquièmement le produit de tête de la zone de concentration est séparé dans la zone de distillation en VCM très pur qui est obtenu en tant que produit de fond de la zone de distillation et en HCl assez pur qui se compose d'HCl et de composants à bouillant plus facilement que le VCM et qui est obtenu en tête de la zone de distillation et

- sixièmement le mélange provenant du fond de la zone de concentration est séparé dans la zone de rectification, étant entendu que dans le fond de la zone de rectification on retire un EDC assez pur comportant tous les composants à bouillant à température plus élevée que le VCM et comportant seulement une très faible teneur en HCl et en VCM, alors qu'en tête de la zone de rectification on obtient un produit qui se compose d'EDC et de HCL et de VCM introduits dans l'amenée ainsi que du HCL et du VCM nouvellement formés par l'instabilité thermique de l'EDC, et est ramené dans la zone de concentration à des fins de retraitement ultérieur.

6. Procédé selon la revendication 1 ou l'une des suivantes **caractérisé en ce qu'**un mélange introduit dans la zone de concentration, débarrassé des matières solides mais ayant une pression relativement basse, est porté à l'aide d'une unité de compression à une pression qui assure un fonctionnement rentable de la zone de concentration.

7. Procédé selon la revendication 1 ou l'une des suivantes **caractérisé en ce que** le fonctionnement de la zone de rectification est assuré aussi bien à une pression inférieure qu'à une pression supérieure à la pression utilisée dans la zone de concentration.

8. Procédé selon la revendication 1 ou l'une des suivantes **caractérisé en ce qu'**un ou plusieurs vaporisateurs de circulation de fond de la zone de distillation sont chauffés avec le produit de fond de la zone de rectification.

9. Procédé selon la revendication 1 ou l'une des suivantes **caractérisé en ce que** le produit de fond de la zone de distillation est amené par une zone d'épuration fine qu'on peut raccorder facultativement, un écoulement de produit très pur étant rectifié dans le fond de la zone d'épuration fine et le produit de tête de la zone d'épuration fine étant recyclé vers la zone de distillation.

10. Procédé selon la revendication 1 ou l'une des suivantes **caractérisé en ce qu'**est mis en oeuvre un mélange gazeux chargé en particules de matières solides, comme par exemple de la suie, obtenu à une température de 480 à 540 °C et à une pression de 0,5 MPa à 3,0 MPA et éventuellement refroidi à une température de 180 à 280 °C dans le cadre d'une récupération de chaleur.

11. Procédé selon les revendications 1 à 6 **caractérisé en ce que** le mélange gazeux mis en oeuvre dans la zone de refroidissement présente une pression inférieure à la pression qui règne dans la zone de rectification et qu'au lieu de la détente dans la première étape du retraitement du fond de refroidissement, le fond de refroidissement est porté avec une pompe à une pression un peu plus élevée que celle qui règne dans la zone de rectification.

12. Procédé selon les revendications 1 à 7 **caractérisé en ce que** le mélange gazeux mis en oeuvre provient d'un procédé de craquage de l'EDC dans lequel on opère avec une vaporisation externe de l'EDC et dans lequel on obtient un écoulement de purge d'EDC, l'écoulement de purge d'EDC étant inclus dans le retraitement du fond de refroidissement et que

- soit il est introduit dans le fond de refroidissement,
- soit il remplace dans la première étape du retraitement du fond de refroidissement l'apport externe de chaleur par alimentation par vaporisation par détente gazeuse.

Fig. 1

**Fig. 2**

EP 1 070 035 B1

Fig. 3